# EUROPÄISCHE PATENTSCHRIFT

(11) **EP 3 524 302 B1**
(45) Veröffentlichungstag und Bekanntmachung des Hinweises auf die Patenterteilung: **06.08.2025**
(21) Anmeldenummer: 18155701.8
(22) Anmeldetag: 08.02.2018
(51) Int. Cl.: A61M 11/02

(54) **AEROSOLERZEUGER**
AEROSOL GENERATOR
GÉNÉRATEUR D'AÉROSOL

(43) Veröffentlichungstag der Anmeldung: 14.08.2019
(73) Patentinhaber: NLI GmbH, 35043 Marburg (DE)
(72) Erfinder: Köhler, Niklas Alexander, 35037 Marburg (DE)
(74) Vertreter: Tergau & Walkenhorst Intellectual Property GmbH

(56) Entgegenhaltungen:
- WO-A1-00/15282
- WO-A1-93/01891
- DE-A1- 10 102 526
- US-A- 5 596 982
- US-A1- 2012 174 916
- US-A1- 2016 256 637

## Beschreibung

Die Erfindung betrifft einen Aerosolerzeuger mit einem Wirkflüssigkeitsbehälter und einer diesem zugeordneten Vernebelungskammer, die mit einem Eingangskanal zur Zuführung von Trägergas und mit einem Auslasskanal zur Abführung von mit aus der Wirkflüssigkeit gewonnenem Aerosol versetztem Trägergas verbunden ist.

Aerosolerzeuger der genannten Art können beispielsweise in so genannten Inhalatoren zum Einsatz kommen, um einen in Form einer Flüssigkeit vorliegenden Wirkstoff oder ein Arzneimittel zu vernebeln oder daraus feine und feinste Tröpfchen zu erzeugen. In Form eines so erzeugten Aerosols kann der Wirkstoff oder das Arzneimittel dann insbesondere mit der Atemluft eines Patienten vermischt und über die Atemluft in den Atemtrakt eingebracht werden. Eine solche Verabreichung von Wirkstoffen oder Arzneimitteln über die Atemluft in den Atemtrakt kann sehr effektiv und wirksam sein, da von der Lunge aus eine schnelle und verlustarme Übertragung in die Blutbahn erfolgen kann.

Aus der WO 2012/079684 A1 ist es zudem bekannt, dass mittels eines derartigen Aerosolerzeugers erzeugte Wirkstoff-Aerosole im Rahmen einer transnasalen Inhalationstherapie verabreicht werden können, wobei bei geeigneter Durchführung, also insbesondere bei vergleichsweise langsamer Verabreichung mit besonders gering gehaltener Tröpfchengröße, eine Deposition des Wirkstoffs vergleichsweise tief in der Lunge und damit effizient am erwünschten Wirkort, nämlich in der Lungenperipherie, möglich ist. Insbesondere für derartige Anwendungen ist es wünschenswert, bei der Vernebelung des Wirkstoffs die Tröpfchengröße der im Aerosolerzeuger erzeugten Tröpfchen besonders genau einstellen und steuern zu können.

Aus der WO 2010/149144 A1 ist ein Aerosolerzeuger der oben genannten Art bekannt, der bei gering gehaltenem Strömungswiderstand für das Atmen eine zuverlässige Erzeugung von Aerosoltröpfchen mit gering gehaltener Tröpfchengröße ermöglichen soll. Dazu umfasst der bekannte Aerosolerzeuger eine im wesentlich zylindrisch ausgestaltete Vernebelungskammer. Das in diese einströmende Trägergas, also insbesondere die Atemluft für den Patienten,wird dabei in der Vernebelungskammer, in der auch die "Beladung" mit dem aus der Wirkflüssigkeit gewonnenem Aerosol erfolgt, mittels einer Leitschaufelanordnung in einen Drall um die Zylinderachse versetzt. Fliehkraftbedingt werden dadurch die vergleichsweise großen und entsprechend schweren Aerosoltröpfchen an die Innenseite dieser Leitschaufeln gedrückt und dort zu Tröpchen agglomeriert und abgeschieden, so dass sie an der Gehäusewand entlang in den Wirkstoffbehälter zurückgeführt werden. Mit dieser Anordnung kann somit die Zuführung unerwünscht großer Aerosoltröpfchen zum Auslasskanal des Aerosolerzeugers begrenzt werden; eine weitergehende Steuerung oder sogar gezielte Einstellung einer gewünschten Tröpfchengröße in der auslassseitig abgegebenen mit Aerosol versetzten Atemluft ist mit diesem System aber nicht möglich.

US2016/256637A1 offenbart einen Dosierinhalator mit einem Ultraschallzerstäuber, der einen Aerosol-Dom umfasst, in dem eine Prallplatte angeordnet ist, und einen Zuluftschlauch, der in den Aerosol-Dom eintritt, um einen Wirbelstrom zu erzeugen. Der Aerosol-Dom ist fest mit einem Abluftrohr und einer konzentrisch zum Aerosol-Dom angeordneten Kuppel verbunden und wird von diesen abgedeckt. Das Abluftrohr steht mit einem Mundstück in Verbindung.

WO00/15282A1 offenbart einen Ultraschallvernebler, der eine Kammer, eine Prallplatte und eine zentrale Rohrstruktur umfasst, die mit einem Förderrohr in Verbindung steht. Die Kammer wird über zwei tangentiale Kanäle mit Druckgas aus einer Vorkammer versorgt. Die Ausrichtung der Kanäle erzeugt eine schnelle, kreisförmige oder wirbelartige Strömung des Gases in der Kammer, die sich mit einem groben Aerosol vermischt, das eine piezoelektrische Scheibe erzeugt.

Der Erfindung liegt daher die Aufgabe zugrunde, einen Aerosolerzeuger der eingangs genannten Art anzugeben, mit dem die Tröpfchengröße der im abgegebenen Trägergas enthaltenen Aerosole besonders gut einstellbar ist und der somit für eine Verwendung gerade im Rahmen einer transnasalen Inhalationstherapie besonders geeignet ist.

Diese Aufgabe wird durch die Merkmale des unabhängigen Anspruchs 1 gelöst.

Vorteilhafte Ausgestaltungen der Erfindung sind Gegenstand der Unteransprüche.

Die Erfindung geht von der Überlegung aus, dass unter Nutzung einer Rotation oder eines Dralls des durch die Vernebelungskammer strömenden, mit Aerosol beladenen Trägergases zur Abscheidung unerwünscht großer Aerosoltröpfchen an der Begrenzungswand die Einstellbarkeit der Tröpfchengröße weiter verbessert werden kann, indem ein zusätzlicher Freiheitsgrad zur Beeinflussung der fliehkraftbedingten Abscheidung bereitgestellt wird. Um dies zu ermöglichen, ist vorgesehen, den Drall oder die Rotation des die Vernebelungskammer durchströmenden Trägergases unabhängig von statischen Einbauten innerhalb der Vernebelungskammer zu erzeugen. Dies ist auf besonders einfache Weise erreichbar, indem der Aerosolerzeuger für eine außermittige Einspeisung des Trägergases, also versetzt zur Rotations- oder Symmetrieachse der Vernebelungskammer, in die Vernebelungskammer ausgelegt ist. Das Trägergas sollte somit in einer Einströmrichtung in die Vernebelungskammer eingespeist werden, die bezogen auf deren Rotationsachse beim Eintritt in die Vernebelungskammer eine tangentiale Komponente aufweist. Damit kann der Drall und damit die Winkelgeschwindigkeit der Rotation des Trägergases innerhalb der Vernebelungskammer durch Einstellung oder Steuerung der Parameter des Trägergases bei der Einströmung, also beispielsweise Volumenstrom und/oder Geschwindigkeit, beeinflusst werden.Insbesondere kann somit das Gas und/oder Aerosol kontrolliert in Rotation versetzt werden.

Vorteilhafterweise ist der Einlasskanal der Vernebelungskammer eingangsseitig mit einer Pumpeneinheit, insbesondere einem Lüfter oder "Blower", verbunden. Die Pumpeneinheit ist dabei vorteilhafterweise hinsichtlich ihrer Förderrate einstellbar, so dass die Einströmrate und/oder -geschwindigkeit des in die Vernebelungskammer einströmenden Trägergases einstellbar ist.Damit kann dann insbesondere auch die Aerosolgröße, d. h. die mittlere Größe der Tröpfchen des Aerosols, reguliert werden.

Die Vernebelung der Wirkflüssigkeit kann dabei mit einem beliebigen Vernebler geeigneter Bauart, beispielsweise einem Düsenvernebler, erfolgen. Erfindungsgemäß ist dazu ein Ultraschallvernebler vorgesehen. Dieser umfasst vorzugsweise in an sich bekannter Bauweise einen Piezokristall, der Ultraschall erzeugt, wobei der Ultraschall auf die Wirkflüssigkeit im Wirkflüssigkeitsbehälter übertragen wird und an deren Oberfläche einen Sprudel bildet, von dem sich feine Tröpfchen als Aerosol absondern.

Bei der Vernebelung der Wirkflüssigkeit, insbesondere mittels Ultraschall, kann oberhalb des Flüssigkeitsspiegels, insbesondere in dem sich bildenden Sprudel, eine Flüssigkeitssäule entstehen. Um deren Höhe mit einfachen Mitteln zu begrenzen und dabei auf besonders einfache Weise bereits eine Abscheidung besonders großer Flüssigkeitströpfchen zu gewährleisten, ist in der Vernebelungskammer dem Wirkflüssigkeitsbehälter gegenüberliegend eine Prallplatte angeordnet. An dieser schlagen sich insbesondere die größten Flüssigkeitströpfchen nieder, so dass sie von der Prallpatte aus in den Wirkflüssigkeitsbehälter zurücktropfen können. Erfindungsgemäß ist dabei der Abstand zwischen Prallplatte und Wirkflüssigkeitsbehälter bzw. der Oberfläche der Wirkflüssigkeit einstellbar, so dass die Höhe der Flüssigkeitssäule und die Tröpfchenabscheidung auf besonders einfache Weise bedarfs- und betriebsweisenabhängig beeinflusst werden können. Insbesondere kann in der als besonders vorteilhaft und eigenständig erfinderisch angesehenen Kombination von einstellbarem Abstand zwischen Prallplatte und Wirkflüssigkeit einerseits mit der einstellbaren Einspeiserate des Trägergases in die Vernebelungskammer die Partikelgröße des entstehenden Aerosols besonders wirksam beeinflusst werden.

Auf besonders einfache Weise ist dies erreichbar, indem die Prallplatte vor der Einmündungsöffnung des Auslasskanals und beabstandet von dieser angeordnet ist.

Eine besonders einfache und damit kostengünstige Bauweise ist erreicht, indem der Auslasskanal von einem Auslassrohr gebildet ist, das in seiner Längsrichtung verschiebbar durch einen Deckelflansch der Vernebelungskammer geführt ist. Insbesondere kann dabei das Auslassrohr mit einem Gewinde in den Deckelflansch eingeschraubt sein, so dass ein Verdrehen des Auslassrohrs im Deckelflansch über das Gewinde in eine Verschiebung in Längsrichtung umgesetzt wird. Besonders bevorzugt ist dabei die Prallplatte endseitig und beabstandet von der Einmündungsöffnung des Auslassrohrs an diesem, beispielsweise über Stege, angebracht, so dass über ein Verschieben des Auslassrohrs im Deckelflansch, beispielsweise mittels Verdrehen, auch die Höhe der Positionierung der Prallplatte oberhalb der Wirkflüssigkeit veränderbar ist. Eine derartige Höhenverstellung, beispielsweise durch Verdrehen, kann dabei manuell erfolgen. Eine erhöhte Präzision ist in einem solchen System aber erreichbar, indem in vorteilhafter Weiterbildung eine automatisierte Höheneinstellung, beispielsweise über einen Schrittmotor, vorgesehen ist. Dieser kann direkt auf die lineare Positionierung der am Auslassrohr montierten Prallplatte einwirken oder aber auch das Verdrehen des Auslassrohrs und somit über das Gewinde die Höhenverstellung bewirken.

Vorteilhafterweise ist der Auslasskanal zumindest im Bereich seiner Einmündung in die Vernebelungskammer mit seiner Längsrichtung im Wesentlichen parallel zur Zylinder- oder Symmetrieachse der Vernebelungskammer ausgerichtet, so dass das mit dem Aerosol beladene Trägergas in einer im Wesentlichen zur Rotationsachse der Vernebelungskammer parallelen Richtung aus dieser abströmt. Bezogen auf das Zylinder-Koordinatensystem der Vernebelungskammer erfolgt die Abströmung des Trägergases somit im Wesentlichen in axialer Richtung und damit orthognal zur tangentialen Richtungskomponente des einströmenden Trägergases. Damit kann über die Strömungsverhältnisse des Trägergases in der Vernebelungskammer der erwünschte Drall besonders wirksam erzeugt und eingestellt werden.

Als Trägergas ist in besonders vorteilhafter Ausgestaltung Sauerstoff oder ein saurstoffhaltiges Gas vorgesehen, so dass das Trägergas auch als Atemgas für den Patienten oder Nutzer besonders gut geeignet ist. Das suaerstoffhaltige Trägergas kann dabei beispielsweise von einer Sauerstoffversorgung oder von einem Sauerstoffkonzentrator aus der Vernebelungskammer zugeführt werden. Um dies zu ermöglichen, ist die Vernebelungskammer und/oder der ihr gasseitig vorgeschaltete Lüfter oder Blower zweckmäßigerweise eingangsseitig mit einer Sauerstoffversorgung oder einem Sauerstoffkonzentrator verbunden.

In weiterer vorteihafter Ausgestaltung ist die Begrenzungswand und/oder der Deckel der Vernebelungskammer mit einer Nachfüllöffnung für die Wirkflüssigkeit versehen, so dass, beispielsweise mit einer Spritze, eine Nachbefüllung des Aerosolerzeugers mit Wirkflüssigkeit möglich ist, ohne dass eine Demontage der Vernebelungskammer erforderlich wäre. In weiterer besonders bevorzugter Ausgestaltung ist die Vernebelungskammer dabei über die Nachfüllöffnung oder über eine geeignete Verbindungsleitung mit einem externen Vorratsbehälter oder einem Reservoir für Wirkflüssigkeit verbunden, so dass die Flüssigkeitshöhe der Wirkflüssigkeit und somit deren Flüssigkeitsspiegel mittels einer geeigneten Nachbefüllung gleichbleibend oder konstant gehalten werden kann.

Die mit der Erfindung erzielten Vorteile bestehen insbesondere darin, dass durch die bezogen auf die Rotationsachse der Vernebelungskammer außermittige Zuführung des Trägergases, insbesondere in Verbindung mit dessen Abführung parallel zur Rotationsachse, ein Drall oder eine Rotation des Gases in der Vernebelungskammer besonders wirkungsvoll eingestellt und auf einfache Weise auch verändert werden kann. Dadurch können die drall- oder rotationsbedingt auf das Gas in der Vernebelungskammer einwirkenden Fliehkräfte gezielt zur größenabhängigen Tröpfchenabscheidung genutzt und bedarfs- und situationsabhängig verändert werden. Damit ist eine gezielte Einflussnahme auf die Tröpfchengröße des im ausströmenden Trägergas mitgeführten Aerosols ermöglicht.

Ein Ausführungsbeispiel der Erfindung wird anhand einer Zeichnung näher erläutert. Darin zeigen:
- FIG. 1: einen Aerosolerzeuger in perspektivischer Ansicht,
- FIG. 2: den Aerosolerzeuger nach FIG. 1 im Längsschnitt,
- FIG. 3: den Aerosolerzeuger nach FIG. 1 in Explosionsdarstellung, und
- FIG. 4: eine alternative Ausführungsform eines Aerosolerzeugers mit externem Vorratsbehälter schematisch im Schnitt.

Gleiche Teile sind in allen Figuren mit denselben Bezugszeichen versehen.

Der Aerosolerzeuger 1 gemäß den Figuren ist zur Erzeugung eines Aerosols aus einem in Form einer Flüssigkeit vorliegenden Wirkstoffs oder Arzneimittels, im folgenden als "Wirkstoffflüssigkeit", und zur Beladung eines Trägergases mit dem erzeugten Aerosol oder Tröpfchennebel vorgesehen. Dazu umfasst der Aerosolerzeuger 1 eine Vernebelungskammer 2 und einen zugeordneten Wirkflüssigkeitsbehälter 4, in dem die zu vernebelnde Wirkflüssigkeit vorgehalten wird. Die Vernebelungskammer 2 weist eine zu ihrer durch den Pfeil 6 angedeuteten Zentralachse im Wesentlichen rotationssymmetrische Bauform auf und ist in radialer Richtung durch eine im Wesentlichen zylindrisch ausgeführte Begrenzungswand 8 begrenzt.

Die Vernebelungskammer 2 ist zur Zuführung von Trägergas, beispielsweise der Atemluft eines Patienten, mit einem Einlasskanal 10 und zur Abführung von mit aus der Wirkflüssigkeit gewonnenem Aerosol versetztem Trägergas mit einem Auslasskanal 12 verbunden.

Der nach oben offen gehaltene und somit mit dem Innenraum der Vernebelungskammer 2 gasseitig verbundene Wirkstoffbehälter 4 umfasst zur Aufnahme des Wirkstoffs oder Arzneimittels eine feste ringförmige Außenwand 14, die im Bodenbereich mit einer den unteren Abschluss bildenden Bodenmembran 16, vorzugsweise gebildet aus Polyvinylchlorid (PVC), verbunden ist. Nach außen und nach unten hin ist der Wirkstoffbehälter 4 in montiertem Zustand umgeben von einem Schallübertragungskörper 18, der seinerseits eine beim Betrieb in der Regel vollständig mit Wasser gefüllte Innenkammer 20 bildet. Die PVC-Membran 16 sorgt dafür, dass sich das zu vernebelnde Medium nicht mit dem Wasser des Basistanks vermischt und möglichweise mit der Verneblermembran in Berührung kommt. Der Schallübertragungskörper 18 sitzt seinerseits in montiertem Zustand auf einem Ultraschallvernebler 22 auf. Dieser umfasst in an sich bekannter Bauweise einen Piezokristall 24. Beim Betrieb des Aerosolerzeugers 1 erzeugt der Piezokristall 24 Ultraschall, der über den Schallübertragungskörper 18 und insbesondere über das in der Innenkammer 20 vorgehaltene Wasser auf die Bodenmembran 16 des Wirkstoffbehälters 4 übertragen und über diese in den Wirkstoff eingekoppelt wird. Somit wird der Ultraschall auf die Wirkflüssigkeit im Wirkflüssigkeitsbehälter 4 übertragen und versetzt diese in Schwingung. Dadurch bildet sich an deren Oberfläche ein Sprudel 26, von dem sich feine Tröpfchen als Aerosol absondern. Diese mehrkomponentige Ausführung des Aerosolerzeugers 1 ermöglicht im Betrieb insbesondere eine deutlich erleichterte Reinigung.

Der Auslasskanal 12 wird von einem Auslassrohr 28 gebildet, das durch einen Deckelflansch 30 der Vernebelungskammer 2 geführt ist. Der Deckelflansch 30 dichtet die Vernebelungskammer 2 nach oben hin ab. Im Bereich der Durchführung ist das Auslassrohr 28 mit einem Außengewinde 32 versehen, das mit einem korrespondierenden Innengewinde im Deckelflansch 30 zusammenwirkt. Über diese Gewindekombination ist das Auslassrohr 28 in den Deckelflansch 30 eingeschraubt, so dass ein Verdrehen des Auslassrohrs 28 im Deckelflansch 30 in eine Verschiebung in Längsrichtung umgesetzt wird. Der Auslasskanal 12 und mit diesem das Auslassrohr 28 sind im Ausführungsbeispiel mit ihrer Längsrichtung im Wesentlichen parallel zur durch den Pfeil 6 angedeuteten Rotations- oder Symmetrieachse der Vernebelungskammer 2 ausgerichtet, so dass das mit dem Aerosol beladene Trägergas in einer im Wesentlichen zur Rotationsachse der Vernebelungskammer 2 parallelen Richtung aus dieser abströmt.

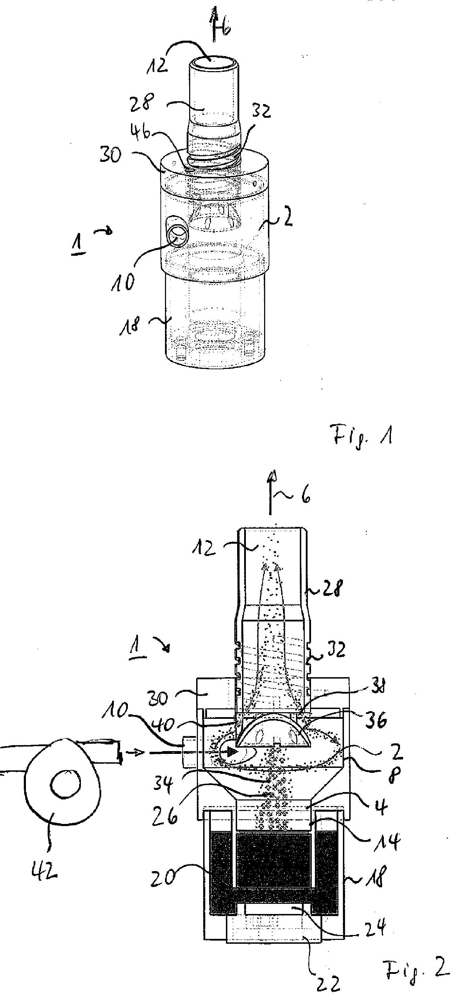

Wie insbesondere der Schnittdarstellung in FIG. 2 entnehmbar ist, entsteht beim Betrieb des Aerosolerzeugers 1 oberhalb des Flüssigkeitsspiegels der Wirkstoffflüssigkeit in dem sich bildenden Sprudel 26 eine Flüssigkeitssäule 34 mit hoher Tröpfchen- oder Flüssigkeitsdichte. Um deren Höhe zu begrenzen und dabei zugleich eine Abscheidung besonders großer Flüssigkeitströpfchen zu ermöglichen, ist in der Vernebelungskammer 2 dem Wirkflüssigkeitsbehälter 4 gegenüberliegend eine Prallplatte 36 angeordnet. An dieser schlagen sich insbesondere die größten Flüssigkeitströpfchen nieder, so dass sie von der Prallpatte 36 aus in den Wirkflüssigkeitsbehälter 4 zurücktropfen können

Die Prallplatte 36 ist endseitig und beabstandet von der Einmündungsöffnung 38 des Auslassrohrs 28 über eine Anzahl von Trägerstegen 40 an diesem angebracht. Dadurch ist über ein Verschieben des Auslassrohrs 28 im Deckelflansch 30, beispielsweise mittels Verdrehen, auch die Höhe der Positionierung der Prallplatte 36 oberhalb der Wirkflüssigkeit veränderbart. Damit sind die Höhe der Flüssigkeitssäule 34 und die Tröpfchenabscheidung an der prallplatte 36 auf besonders einfache Weise bedarfs- und betriebsweisenabhängig beeinflussbar.

Der Aerosolerzeuger 1 ist dafür ausgelegt, die Tröpfchengröße der im abgegebenen Trägergas enthaltenen Aerosole besonders gut einstellen zu könenn, so dass insbesondere eine Verwendung im Rahmen einer transnasalen Inhalationstherapie besonders gut ermöglicht ist. Dazu ist der Aerosolerzeuger 1 für eine Strömungsführung des Trägergases in der Verneblerkammer 2 ausgelegt, bei der das Trägergas auf einer spiralförmigen Strömungsbahn geführt und einem aufgeprägten Drall ausgesetzt wird. Das Design des Vemeblers oder Aerosolerzeugers 1 basiert dabei auf dem Konzept, eine möglichst kleine Aerosol-Partikelgröße über eine Partikeltrennung mittels Zentrifugalkraft zu erreichen. Durch einen geeignet zugeführten Luftstrom innerhalb des zylindrischen Vemeblergehäuses soll es zu einer Kollision von massereichen Aerosolpartikeln an der Vemebler-Innenwand in Abhängigkeit des Flusses und der Partikelgröße kommen. An den Auslasskanal 12 gelangen somit bevorzugt nur noch vergleichsweise kleinere Aerosolpartikeln, welche für die transnasale Inhalationstherapie von Bedeutung sind.

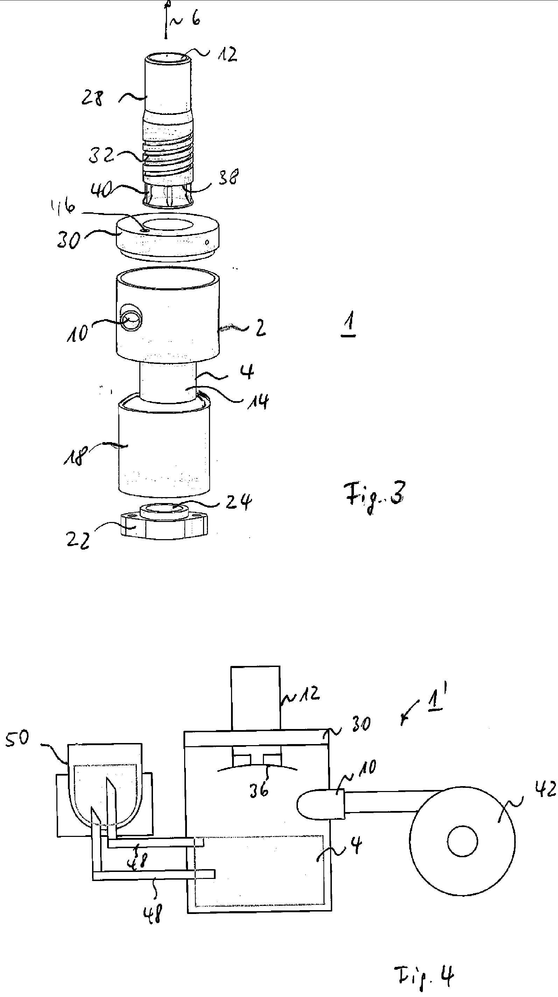

Um die gewünschte Drall- oder Rotationserzeugung auf besonders einfache Weise zu ermöglichen, ist eine geeignet Zuführung der Trägerluft in die Vernebelungskammer 2, nämlich einerseits eine außermittige Zuführung und andererseits eine Zuführung unter geeignet gweähltem Einspeisedruck, vorgesehen. Dazu ist der Einlasskanal 10 im Bereich seiner Einmündungsstelle in die Vernebelungskammer 2 derart außermittig positioniert und orientiert, dass seine Längsachse im Bereich der Einmündungsstelle relativ zur durch den Pfeil 6 angedeuteten Rotationsachse der Vernebelungskammer 2 versetzt ist und die Rotationsachse nicht schneidet. Diese Anordnung wird insbesondere in den perspektivischen Darstellungen in den Figuren 1 und 3 deutlich. Zudem ist, wie dies der Schnittzeichnung in FIG. 2 entnehmbar ist, der Einlasskanal 10 eingangsseitig mit einer Pumpeneinheit 42 verbunden, über die die Einströmrate und/oder -geschwindigkeit des in die Vernebelungskammer 2 einströmenden Trägergases einstellbar ist. Als Pumpeneinheit 42 ist im Ausführungsbeispiel ein geeignet gewählter Lüfter oder "Blower" vorgesehen.

Durch diese Positionierung des Einlasskanals 10, ggf. in Kombination mit der einstellbaren Zuführrate des Trägergases über die Pumpeneinheit 42, wird das in die Vernebelungskammer 2 einströmende Trägergas in den gewünschten Drall um die Rotationsachse versetzt. Fliehkraftbedingt werden dadurch die vergleichsweise großen und entsprechend schweren Aerosoltröpfchen an die innere Begrenzungswand 8 gedrückt und dort abgeschieden, so dass sie an der Gehäusewand entlang und über die im unteren Bereich abgeschrägte Wand 44 der Vernebelungskammer 2 in den Wirkflüssigkeitsbehälter 4 zurückgeführt werden. Der Fluss (auch Volumenstrom genannt), welcher das Aerosol in der Vemebelungskammer 2 zu einer kreisenden Bewegung beschleunigt , wird in der Pumpeneinheit 42 erzeugt, wobei über die Änderung der Drehgeschwindigkeit des Lüfters/Blowers der erzeugte Fluss reguliert wird.

Der Deckelflansch 30 der Vernebelungskammer 2 ist mit einer Nachfüllöffnung 46 für die Wirkflüssigkeit versehen, so dass, beispielsweise mit einer Spritze, eine Nachbefüllung des Aerosolerzeugers 1 mit Wirkflüssigkeit möglich ist, ohne dass eine Demontage der Vernebelungskammer 2 erforderlich wäre. Alternativ oder zusätzlich kann die Nachfüllöffnung 46 natürlich auch in der Begrenzungswand 8 angeordnet sein.

Im alternativen Ausführungsbeispiel gemäß FIG. 4 ist der Wirkflüssigkeitsbehälter 4 des Aerosolerzeugers 1' über eine Anzahl von Medienleitungen 48 mit einem externen Reservoir oder Vorratsbehälter 50 für Wirkflüssigkeit verbunden. Über diesen kann auch während des Betriebs des Aerosolerzeugers 1' eine bedarfsweise Nachbefüllung des Wirkflüssigkeitsbehälters 4 erfolgen, wobei insbesondere der Flüssigkeitsspiegel im Wirkflüssigkeitsbehälter 4 kontrolliert und beispielsweise konstant gehalten werden kann.

### Bezugszeichenliste

- 1: Aerosolerzeuger
- 2: Vernebelungskammer
- 4: Wirkflüssigkeitsbehälter
- 6: Pfeil
- 8: Begrenzungswand
- 10: Einlasskanal
- 12: Auslasskanal
- 14: Außenwand
- 16: Bodenmembran
- 18: Schallübertragungskörper
- 20: Innenkammer
- 22: Ultraschallvernebler
- 24: Piezokristall
- 26: Sprudel
- 28: Auslassrohr
- 30: Deckelflansch
- 32: Außengewinde
- 34: Flüssigkeitssäule
- 36: Prallplatte
- 38: Einmündungsöffnung
- 40: Trägersteg
- 42: Pumpeneinheit
- 44: Wand
- 46: Nachfüllöffnung
- 48: Medienleitung
- 50: Vorratsbehälter

## Patentansprüche

1. Aerosolerzeuger (1) mit einem Wirkflüssigkeitsbehälter (4) für eine Wirkstoffflüssigkeit, dem einerseits ein Ultraschallvernebler (22) und andererseits eine Vernebelungskammer (2) zugeordnet sind, wobei in der Verneblungskammer (2) mittels des Ultraschallverneblers (22) oberhalb des Flüssigkeitsspiegels der Wirkstoffflüssigkeit ein Sprudel (26) der Wirkstoffflüssigkeit erzeugbar ist, von dem sich feine Tröpfchen als Aerosol absondern, wobei die Vernebelungskammer (2) mit einem Einlasskanal (10) zur Zuführung von Trägergas und mit einem Auslasskanal (12) zur Abführung von mit aus der Wirkflüssigkeit gewonnenem Aerosol versetztem Trägergas verbunden ist und eine im Wesentlichen rotationssymmetrisch ausgeführte Begrenzungswand (8) aufweist, wobei der Einlasskanal (10) im Bereich seiner Einmündungsstelle in die Verneblerkammer (2) derart positioniert und orientiert ist, dass seine Längsachse im Bereich der Einmündungsstelle relativ zur Rotations- oder Symmetrieachse der Vernebelungskammer (2) versetzt ist und die Rotations- oder Symmetrieachse nicht schneidet,
wobei der Einlasskanal (10) durch die Begrenzungswand (8) hindurch in die Vernebelungskammer (2) derart einmündet, dass das Trägergas in der Vernebelungskammer (2) auf einer spiralförmigen Strömungsbahn geführt und einem aufgeprägten Drall ausgesetzt wird,
wobei in der Vernebelungskammer (2) dem Wirkflüssigkeitsbehälter (4) gegenüberliegend eine Prallplatte (36) angeordnet ist,
der Auslasskanal (12) von einem Auslassrohr (28) gebildet ist, das in seiner Längsrichtung verschiebbar durch einen Deckelflansch (30) der Vernebelungskammer (2) geführt ist, und die Prallplatte (36) endseitig und beabstandet von der Einmündungsöffnung (38) des Auslassrohrs (28) über eine Anzahl von Trägerstegen (40) an diesem angebracht ist, so dass über ein Verschieben des Auslassrohrs (28) im Deckelflansch (30) auch die Höhe der Positionierung der Prallplatte (36) oberhalb der Wirkflüssigkeit veränderbar ist.

2. Aerosolerzeuger (1) nach Anspruch 1, dessen Einlasskanal (10) eingangsseitig mit einer Pumpeneinheit (42) verbunden ist, über die die Einströmrate und/oder -geschwindigkeit des in die Vernebelungskammer (2) einströmenden Trägergases einstellbar ist.

3. Aerosolerzeuger (1) nach einem der Ansprüche 1 bis 2, wobei das Auslassrohr (28) mittels Verdrehen in dem Deckenflansch (30) verschiebbar ist.

4. Aerosolerzeuger (1) nach Anspruch 3, wobei im Bereich der Durchführung durch den Deckenflansch (30) das Auslassrohr (28) mit einem Außengewinde (32) versehen ist, das mit einem korrespondierenden Innengewinde im Deckelflansch (30) zusammenwirkt, und über diese Gewindekombination das Auslassrohr (28) in den Deckelflansch (30) eingeschraubt ist, so dass ein Verdrehen des Auslassrohrs (28) im Deckelflansch (30) in eine Verschiebung in Längsrichtung umgesetzt wird.

5. Aerosolerzeuger (1) nach einem der Ansprüche 1 bis 4, bei dem die Begrenzungswand (8) und/oder der Deckelflansch (30) der Vernebelungskammer (2) eine Anzahl von Nachfüllöffnungen (46) für die Wirkflüssigkeit aufweist.

## Claims

1. An aerosol generator (1) with an active-liquid container (4) for an active-ingredient liquid, to which an ultrasonic nebulizer (22) on the one hand and a nebulization chamber (2) on the other hand are assigned, wherein, in the nebulization chamber (2), a fizzing (26) of the active-ingredient liquid can be generated by means of the ultrasonic nebulizer (22) above the liquid level of the active-ingredient liquid, from which fine droplets are discharged as an aerosol, wherein the nebulization chamber (2) is connected to an inlet channel (10) for the supply of carrier gas and to an outlet channel (12) for the discharge of carrier gas mixed with aerosol obtained from the active liquid, and the nebulization chamber (2) comprises an essentially rotationally symmetrical boundary wall (8), wherein the inlet channel (10) is positioned and orientated in the region of its point of entry into the nebulizer chamber (2) in such a way that its longitudinal axis in the region of the point of entry into the nebulizer chamber (2) is relative to the rotational axis or symmetry of the nebulization chamber (2) and does not intersect the rotational axis or symmetrical axis,
wherein
the inlet channel (10) flows through the boundary wall (8) into the nebulization chamber (2) in such a way that the carrier gas in the nebulization chamber (2) is guided on a spiral flow path and exposed to an imparted swirl,
wherein, in the nebulization chamber (2), a baffle plate (36) is arranged opposite the active-liquid container (4),
the outlet channel (12) is formed by an outlet pipe (28) which is guided in its longitudinal direction by a cover flange (30) of the nebulization chamber (2), and the baffle plate (36) is attached to this at the end of the end and at a distance from the outlet opening (38) of the outlet pipe (28) via a number of carrier bars (40) so that, by shifting the outlet pipe (28) in the cover flange (30), the height of the positioning of the baffle plate (36) above the active liquid can also be changed.

2. The aerosol generator (1) according to Claim 1, the inlet channel (10) of which is connected on the input side to a pump unit (42) by means of which the inflow rate and/or velocity of the carrier gas flowing into the nebulization chamber (2) is adjustable.

3. The aerosol generator (1) according to any one of Claims 1 to 2, wherein the outlet pipe (28) can be moved by twisting in the cover flange (30).

4. The aerosol generator (1) according to Claim 3, wherein, in the area of the penetration through the cover flange (30), the outlet pipe (28) is provided with an external thread (32) which interacts with a corresponding internal thread in the cover flange (30), and, via this thread combination, the outlet pipe (28) is screwed into the cover flange (30) so that a twisting of the outlet pipe (28) in the cover flange (30) is transposed into a shift in the longitudinal direction.

5. The aerosol generator (1) according to any one of the Claims 1 to 4, wherein the boundary wall (8) and/or the cover flange (30) of the nebulization chamber (2) comprises a number of refill openings (46) for the active liquid.

## Revendications

1. Générateur d'aérosol (1) comprenant un réservoir de liquide actif (4) pour un principe actif liquide, auquel sont associés d'une part un nébuliseur à ultrasons (22) et d'autre part une chambre de nébulisation (26), une bulle (2) du principe actif liquide pouvant être générée dans la chambre de nébulisation (2) au-dessus du niveau du principe actif liquide, dont de fines gouttelettes sont détachées sous forme d'aérosol, la chambre de nébulisation (2) étant reliée à un canal d'entrée (10) pour l'amenée du gaz porteur et à un canal de sortie (12) pour l'évacuation du gaz porteur mélangé à l'aérosol obtenu à partir du liquide actif et présentant une paroi de limitation (8) sensiblement symétrique en rotation, le canal d'entrée (10) étant positionné et orienté dans la zone de son point d'embouchure dans la chambre de nébulisation (2) de telle sorte que son axe longitudinal dans la zone du point d'embouchure soit décalé par rapport à l'axe de rotation ou de symétrie de la chambre de nébulisation (2) et ne coupe pas l'axe de rotation ou de symétrie,
le canal d'entrée (10) dans la chambre de nébulisation (2) débouchant à travers la paroi de délimitation (8) de telle sorte que le gaz porteur se trouvant dans la chambre de nébulisation (2) est guidé sur une trajectoire d'écoulement en spirale et est exposé à un tourbillon prononcé,
dans la chambre de nébulisation (2), le réservoir de liquide actif (4) étant disposé en face d'un déflecteur (36), le canal de sortie (12) étant formé par un tuyau de sortie (28) qui est guidé de manière à pouvoir coulisser dans sa direction longitudinale par une bride de recouvrement (30) de la chambre de nébulisation (2), et le déflecteur (36) étant à l'extrémité et espacé de l'ouverture d'embouchure (38) du tuyau de sortie (28) par un certain nombre d'entretoises de support (40) fixées à cette bride de sorte que la hauteur de positionnement du déflecteur (36) au-dessus du liquide actif peut également être modifiée par déplacement du tuyau de sortie (28) de la bride de recouvrement (30).

2. Générateur d'aérosol (1) selon la revendication 1, dans lequel le canal d'entrée (10) est relié côté entrée à une unité de pompage (42) par l'intermédiaire de laquelle le débit et/ou la vitesse d'entrée du gaz porteur s'écoulant dans la chambre de nébulisation (2) est réglable.

3. Générateur d'aérosol (1) selon l'une quelconque des revendications 1 à 2, dans lequel le tuyau de sortie (28) peut coulisser par torsion dans la bride de recouvrement (30).

4. Générateur d'aérosol (1) selon la revendication 3, dans lequel le tuyau de sortie (28) est pourvu, dans la zone du passage à travers la bride de recouvrement (30), d'un filetage extérieur (32) qui coopère avec un filetage intérieur correspondant dans la bride de recouvrement (30) et, par l'intermédiaire de cette combinaison filetée, le tuyau de sortie (28) est vissé dans la bride de recouvrement (30) de sorte qu'une rotation du tuyau de sortie (28) dans la bride de recouvrement (30) se traduit par un déplacement dans le sens longitudinal.

5. Générateur d'aérosol (1) selon l'une quelconque des revendications 1 à 4, dans lequel la paroi de délimitation (8) et/ou la bride de recouvrement (30) de la chambre de nébulisation (2) comporte plusieurs ouvertures de remplissage (46) pour le liquide actif.
